(19) Europäisches Patentamt
European Patent Office
Office européen des brevets

(11) Veröffentlichungsnummer: **0 248 356**
**B1**

(12) # EUROPÄISCHE PATENTSCHRIFT

(45) Veröffentlichungstag der Patentschrift: **27.06.90**

(51) Int. Cl.[5]: **C07D 221/14,** D06L 3/12

(21) Anmeldenummer: **87107792.1**

(22) Anmeldetag: **29.05.87**

(54) **Schwefelsäureester-Gruppen enthaltende Naphthalimide, Verfahren zu deren Herstellung und deren Verwendung.**

(30) Priorität: **02.06.86 DE 3618458**

(43) Veröffentlichungstag der Anmeldung:
**09.12.87 Patentblatt 87/50**

(45) Bekanntmachung des Hinweises auf die Patenterteilung:
**27.06.90 Patentblatt 90/26**

(84) Benannte Vertragsstaaten:
**CH DE FR GB IT LI**

(56) Entgegenhaltungen:
**DE-A-2 944 867**
**DE-B-1 445 961**
**DE-B-1 470 051**
**GB-A-2 127 015**

(73) Patentinhaber: **HOECHST AKTIENGESELLSCHAFT, Postfach 80 03 20, D-6230 Frankfurt am Main 80(DE)**

(72) Erfinder: **Schinzel, Erich, Dr., Ostpreussenstrasse 43, D-6238 Hofheim am Taunus(DE)**
Erfinder: **Martini, Thomas, Dr., Odenwaldestrasse 5, D-6233 Kelkheim (Taunus)(DE)**

EP 0 248 356 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bereits bekannt, Sulfonsäuregruppen enthaltende Naphthalimide als optische Aufheller zu verwenden. So werden zum Beispiel in dem CS-Patent 185 273 Salze des N-(β-Sulfoethyl)-4-methoxy-naphthalimids als optische Aufheller beschrieben.

Generell bekannt ist auch die Verwendung von N-Alkyl-4-alkoxy-naphthalsäureimiden als optische Aufheller.

Gegenstand der vorliegenden Erfindung sind Verbindungen der allgemeinen Formel (1),

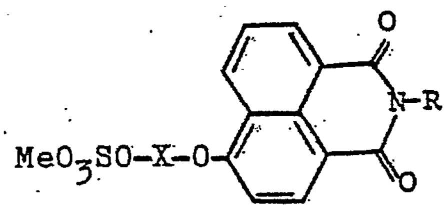

(1)

worin R Wasserstoff, $C_1$–$C_6$-Alkyl, $C_1$–$C_6$-Alkoxy-$C_1$–$C_6$-alkyl, Cyclohexyl, Methylcyclohexyl, Benzyl, Phenylethyl, Phenyl, $C_1$–$C_6$-Alkylphenyl, Xylyl,
X eine $C_2$–$C_7$-Alkylengruppe oder ein Brückenglied der Formel $-X^1-(OX^2)_n-$, worin $X^1$ und $X^2$ $C_2H_4$ oder $C_3H_7$ und n 1 oder 2 bedeuten und
Me ein Proton, ein Alkalimetallkation oder ein $NH_2$ $R^1R^2$Kation, wobein $R^1$ und $R^2$ für Wasserstoff, $C_1$–$C_6$-Alkyl oder Hydroxy-$C_1$–$C_6$-Alkyl stehen, bedeuten.

Von besonderer Bedeutung sind Verbindungen der Formel (1), worin R Wasserstoff, $C_1$–$C_4$-Alkyl oder Benzyl, X $C_2$–$C_5$-Alkylen und Me ein Proton, ein Alkalimetallkation oder ein $-NH_2R^1R^2$ Kation, wobei $R^1$ und $R^2$ für Wasserstoff $C_1$–$C_2$-Alkyl oder $C_1$–$C_2$-Hydroxyalkyl stehen, bedeuten.

Alkyl- und Alkoxygruppen enthalten vorzugsweise 1 bis 4 C-Atome.

Zur Herstellung der Verbindungen der Formel (1) kann man die Hydroxyverbindungen der allgemeinen Formel (2),

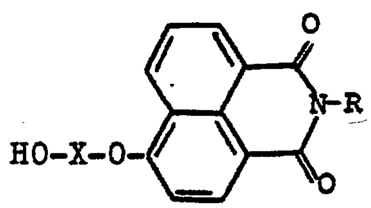

(2)

in welcher R und X die oben angegebene Bedeutung haben, bei Temperaturen von 0-30°C, vorzugsweise 5-10° in konz. Schwefelsäure eintragen und bis zur Bildung einer homogenen Lösung 1 bis 4 Stunden bei dieser Temperatur verrühren. Zur Aufarbeitung wird auf Eis/Wasser gegossen, mit konz. Natronlauge neutralisiert, nach Eiskühlung des auskristallisierte Natriumsulfat-Dekahydrat abgesaugt und mit Eiswasser nachgewaschen. Im Filtrat wird z.B. durch Aussalzen mit Kochsalz das Na-Salz der Schwefelsäureester (1) abgeschieden, abgesaugt und mit Kochsalzlösung sulfatfrei gewaschen.

Die Verbindungen der allgemeinen Formel (2) können durch Kondensation des 4-Chlor-naphthalsäureanhydrids mit 1 Mol eines Amins ($R-NH_2$) in einem niederen Alkohol bei Temperaturen von 50-80°C während 4 bis 10 Stunden und anschließender Umsetzung des erhaltenen 4-Chlor-naphthalimids (3)

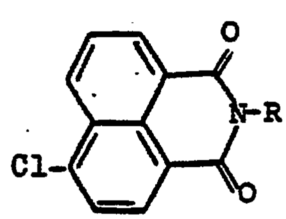

(3)

mit einer Lösung eines Mononatriumsalzes der Formel NaO-X-OH im Überschuß des entsprechenden Diols bei 100 bis 110°C in 3 bis 10 Stunden hergestellt weren. Diese Lösung kann erhalten werden durch Verrühren von 1 Mol einer methanolischen Natriummethylatlösung mit überschüssigem Diol bei Raumtemperatur und anschließendes Abziehen von Methanol im Vakuum bei 60-70°C.

Als Amine $R-NH_2$ können beispielsweise eingesetzt werden:
Ammoniak, Methylamin, Ethylamin, n-Propylamin, Isopropylamin, 3-Methoxy-propylamin, n-Butylamin, Isobutylamin, tert.-Butylamin, n-Hexylamin, Cyclohexylamin, 4-Aminohexahydrotoluol, Anilin, 3-Amino-toluol, 4-Amino-toluol, Benzylamin, 1-Amino-4-ethyl-benzol, p-tert. Butylanilin, β-Phenylethylamin, 4-Amino-1,2-xylol, 4-Amino-1,3-xylol, 4-Amino-1-isopropyl-benzol.

Als Diole HO-X-OH können beispielsweise eingesetzt werden:

Ethylenglykol, Propan-diol-(1,2), Propan-diol-(1,3), n-Butan-diol-(1,3), n-Butan-diol-(1,4), n-Butan-diol(2,3), 2,2-Dimethyl-propan-diol-(1,3), 2,2'-Diethyl-propan-diol-(1,3), Pentandiol-(1,5), n-Hexan-diol-(2,5), n-Hexandiol-(1,6) 2,3-Dimethyl-n-butandiol-(2,3), Diethylenglykol und Triethylenglykol.

Die erfindungsgemäßen neuen Verbindungen zeigen in gelöstem oder feinverteiltem Zustande eine mehr oder weniger ausgeprägte Fluoreszenz. Sie werden zum optischen Aufhellen oder veschiedenen synthetischen, halbsynthetischen oder natürlichen organischen Materialien verwendet.

Hierfür seien beispielsweise, ohne daß durch die nachfolgende Übersicht irgendeine Beschränkung hierauf ausgedrückt werden soll, die folgenden Gruppen von organischen Materialien genannt:

1) Polymerisationsprodukte, beispielsweise Polymerisate auf Basis von α,β-ungesättigten Carbonsäuren oder Derivaten solcher Carbonsäuren, insbesondere von Acrylverbindungen, von Olefin- Kohlenwasserstoffen und Polymerisate auf Basis von Vinyl- und Vinyliden-Verbindungen,

2) Polymerisationsprodukte die durch Ringöffnung erhältlich sind, z.B. Polyamide vom Polycaprolactam-Typ, ferner Polymere, die sowohl über Polyaddition als auch Polykondensation erhältlich sind, wie Polyether der Polyacetale,

3) Polykondensationsprodukte oder Vorkondensate auf Basis bi- oder polyfunktioneller Verbindungen mit kondensationfähigen Gruppen, deren Homo- und Mischkondensationsprodukte, wie beispielsweise Polyester, insbesondere gesättigte (z.b. Ethylenglykolterephtalsäure-Polyester), unverzweigte sowie verzweigte (auch auf Basis höherwertiger Alkohole, wie z.B. Alkydharze) Polyester, Polyamide (z.B. Hexamethylendiamin-adipat).

4) Halbsynthetische organische Materialien, z.B. Celluloseester verschiedener Veresterungsgrade (sogenanntes 2 1/2-Acetat, Triacetat), Celluloseether oder regenerierte Cellulose.

5) Natürliche organische Materialien animalischen oder vegetabilischen Ursprungs, beispielsweise auf Basis von Cellulose oder Proteinen wie Baumwolle, Wolle, Leinen und Seide.

Bevorzugte organische Materialien sind solche aus Polyamid und Wolle.

Die optisch aufzuhellenden organischen Materialien können den verschiedenartigsten Verarbeitungszuständen (Rohstoffe, Halbfabrikate oder Fertigfabrikate) angehören.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundstoffe, Gewirke sowie als Papiere, Pappen oder Papiermasen vorliegen.

Den erfindungsgemäß anzuwendenden Verbindungen kommt u.a. Bedeutung für die Behandlung von textilen organischen Materialien, insbesondere textilen Geweben, zu.

Sofern Fasern, welche als Stapelfasern oder Endlosfäden, in Form von Strängen, Geweben, Gewirken, Vliesen, beflockten Substraten oder Verbundstoffen vorliegen können, erfindungsgemäß optisch aufzuhellen sind, geschieht dies mit Vorteil in wäßrigem Medium, worin die betreffenden Verbindungen gelöst oder in feinverteilter Form (Suspensionen, sogenannten Mikrodispersionen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. Die Behandlung wird üblicherweise bei Temperaturen von etwa 20 bis 140°C, beispielsweise bei Siedetemperatur des Bades oder in deren Nähe (etwa 90 °C), durchgeführt. Für die erfindungsgemäße Veredlung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die neuen optischen Aufhellmittel gemäß vorliegender Erfindung können ferner den Materialien vor oder während deren Verformung zugesetzt bzw. einverleibt werden. So kann man sie beispielsweise bei der Herstellung von Filmen, Folien (z.B. Einwalzen in Polyvinylchlorid in der Hitze) oder Formkörpern der Pressmasse oder Spritzgußmasse beifügen.

Die neuen optischen Aufhellmittel gemäß vorliegender Erfindung können beispielsweise auch in folgenden Anwendungsformen eingesetzt werden:

1) Mischungen mit Farbstoffen (Nuancierung) oder Pigmenten oder als Zusatz zu Färbebädern, Druck-, Ätz- oder Reservepasten. Ferner auch zur Nachbehandlung von Färbungen, Drucken oder Ätzdrucken,

2) in Mischungen mit sogenannten "Carriern", Netzmitteln, Weichmachern, Quellmitteln, Antioxydantien, Lichtschutzmitteln, Hitzestabilisatoren und besonders chemischen Bleichmitteln (Chlorit-Bleiche, Bleichbäder-Zusätze),

3) in Mischung mit Vernetzern, Appreturmitteln (z.B. Stärke oder synthetischen Appreturen) sowie in Kombination mit den verschiedensten Textilveredlungsverfahren, insbesondere Kunstharzausrüstungen, ferner Flammfest-, Weichgriff-, Schmutzablöse- oder Antistatisch-Ausrüstungen oder antimikrobiellen Ausrüstungen,

4) in Kombination mit anderen, optisch aufhellend wirkenden Substanzen.

In gewissen Fällen werden die Aufheller durch eine Nachbehandlung zur vollen Wirkung gebracht. Diese kann beispielsweise eine chemische (z.B. Säure-Behandlung), eine thermische (z.B. Hitze) oder eine kombinierte chemisch/thermische Behandlung darstellen.

Die Menge der erfindungsgemäß zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,0001 Gewichtsprozent, kann ein deutlicher und haltbarer Effekt erzielt werden. Für die meisten praktischen Belange sind vorzugsweise Mengen zwischen 0,01 und 0,5 Gewichtsprozent von Interesse.

Die erfindungsgemäßen Aufheller könne auch in Mischung mit anderen Aufhellern eingesetzt werden. Da einige der erfindungsgemäßen Aufheller Aufhelleffekte mit grüner Nuance bewirken, ist es besonders vorteilhaft, sie gemeinsam mit Aufhellern zu verwenden, die rotstichige Aufhelleffekte zeigen.

Die neuen optischen Aufheller haben den besonderen Vorteil, daß sie auch bei Gegenwart von Aktivchlorspendern, wie z.B. Hypochlorit, wirksam sind und ohne wesentliche Einbuße der Effekte in Waschbädern mit nichtionogenen Waschmitteln, z.b. Alkylphenolpolyglykolethern, verwendet werden können.

In den Beispielen sind Teile, soweit nicht anders angegeben, immer Gewichtsteile und Prozente immer Gewichtsprozente. Schmelz- und Siedepunkte sind, sofern nicht anders vermerkt, unkorrigiert.

Herstellungsbeispiele

4-Chlor-naphthalimide (Tabelle 1)

255,6 g 4-Chlor-naphthalsäure-anhydrid (91 %ig) werden in einem 5 l Rührautoklaven in 2,4 l Methanol suspendiert, mit 75,94 g Methylaminlösung (40,9 %ig) versetzt und 4 Stunden bei 70°C verrührt. Nach dem Erkalten wird bei Raumtemperatur abgesaugt und das Nutschgut mit Methanol bis zum farblosen Ablauf gewaschen. Man trocknet bei 60°C im Vakuum. Es werden 223,8 g der Verbindung (11) erhalten, die nach Umlösung in Toluol einen konstanten Schmelzpunkt von 172-175° zeigt.

Im analoger Weise werden unter Verwendung von Ethanol oder n-Butanol als Lösungsmittel und bei Reaktionszeiten bis 9 Stunden die in der Tabelle 1 angeführten 4-Chlor-naphthalimide erhalten.

Tabelle 1

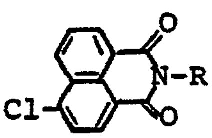

| Nr. | R | Schmelzpunkt °C | Reinigungsmittel |
|---|---|---|---|
| (11) | $-CH_3$ | 172-175 | Toluol |
| (12) | $-(CH_2)_3CH_3$ | 93-95 | Ethanol |
| (13) | $-(CH_2)_3OCH_3$ | 84-88 | Essigsäureethylester |
| (14) | 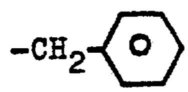 | 175-177 | Toluol |
| (15) | 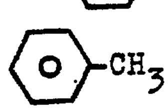 | 248-250 | Toluol |

4-Hydroxyalkoxy-naphthalimide (Tabelle 2)

Unter Ausschluß von Luftfeuchtigkeit werden in einem Rührgefäß 160 ml Ethylenglykol vorgelegt und 18,12 g einer methanolischen Natriummethylatlösung (29,8 %ig) zugetropft. Man verrüht 2 Stunden bei Raumtemperatur und destilliert anschließend das Methanol bei 60-70°C im Wasserstrahlvakuum ab. Bei Raumtemperatur werden 28,77 g des N-n-Butyl-4-chlor-naphthalimids (12) eingetragen. Das hellgelbe Reaktionsgemisch wird auf 100°C angeheizt, 3 Stunden bei 100-105°C verrührt, auf Raumtemperatur erkalten gelassen und mit 160 ml Wasser verdünnt. Nach dem Absaugen wird der Nutschkuchen mit Wasser neutral und frei von Chlorionen gewaschen. Man trocknet bei 60°C im Vakuum. Es werden 20,7 g der Verbindung (22) erhalten, die nach Umlösung ins Toluol bei 127-129 konstant schmilzt.

Die in der Tabelle 2 angegebenen Verbindungen können in analoger Weise hergestellt werden, wobei in einzelnen Fällen die Reaktionszeit bis auf 10 Stunden ausgedeht werden muß.

Tabelle 2

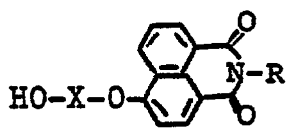

| Nr. | R | X | Schmelzpunkt °C | Reinigungsmittel |
|---|---|---|---|---|
| (21) | $-CH_3$ | $-CH_2CH_2-$ | 204-207 | Benzoesäuremethylester |
| (22) | $-(CH_2)_3CH_3$ | $-CH_2CH_2-$ | 127-129 | Toluol |
| (23) | $-(CH_2)_3OCH_3$ | $-CH_2CH_2-$ | 144-146 | Toluol |
| (24) | 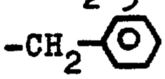 | $-CH_2CH_2-$ | 157-159 | Essigsäureethylester |
| (25) | 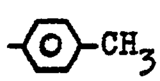 | $-CH_2CH_2-$ | 278-280 | Benzoesäuremethylester |
| (26) | $-CH_3$ | $-(CH_2)_3-$ | 164-167 | Toluol |
| (27) | $-CH_3$ | $-(CH_2)_4-$ | 166-168 | Chlorbenzol |
| (28) | $-CH_3$ | $-CH_2C(CH_3)_2CH_2-$ | 212-214 | Chlorbenzol |
| (29) | $-CH_3$ | $-CH_2CH_2OCH_2CH_2-$ | 135-137 | Essigester |

Schwefelsäureestersalze der 4-Hydroxyalkoxy-naphthalimide

In 33 ml konz. Schwefelsäure (96 %ig) werden unter Rührung bei 5-10° in 30 Min. 14,26 g des 4-(3-Hydroxy-propoxy)-N-methyl-naphthalimids (26) eingetragen. Man rührt bei dieser Temeperatur 3 Stunden nach, bis sich eine bräunlich-gelbe, homogene Lösung gebildet hat und sich eine entnommene kleine Probe klar in Wasser löst. Anschließend wird auf 260 g Eis/Wasser gegeben und unter Kühlung mit konz. Natronlauge neutral gestellt. Nach 1 Stunde wird das ausgefallene Natriumsulfat-Dekahydrat bei 10° abgesaugt und mit Eiswasser bis zum farblosen Ablauf nachgewaschen. Das Filtrat (400 ml) wird mit 80 g Natriumchlorid versetzt, 2 Stunden bei Raumtemperatur verrührt, abgesaugt und das Nutschgut mit 20 %iger NaCl-Lösung frei von Sulfationen gewaschen. Man trochnet bei 60° im Vakuum. Es werden 19,0 g des folgenden Schwefelsäureestersalzes,

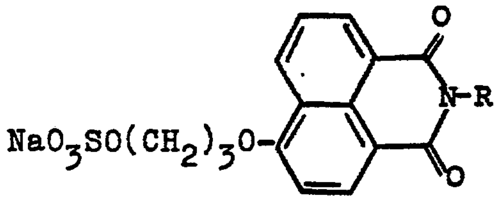

mit einem Gehalt von 7,5 % NaCl und 0,8 % Wasser, erhalten.

In analoger Weise werden die in Tabelle 2 angegebenen 4-Hydroxy-alkoxy-naphthalimide in ihre Schwefelsäureestersalze verwandelt. Die so hergestellten Verbindungen sind in Tabelle 3 aufgeführt.

Anwendungsbeispiele

Beispiel 1

10 g Polyamid-6-Gewebe wurden im Laborfärbeapparat aufgehellt mit den in Tabelle 3 aufgeführten Verbindungen.

Flottenzusammensetzung:

Flotte 1:20
0,25 % Optische Aufheller, bezogen auf Warengewicht, 100 % Wirksubstanz
2,0 g/l Natriumacetat
1,0 ml/l Essigsäure
0,5 g/l Nonylphenolpolyglykolether

Mit der Ware wurde bei ca. 40 °C in die Flotte eingegangen, die Becher eingesetzt und der Apparat dann in etwa 30 Minuten auf 85 °C gebracht. Bei dieser Temperatur wurde weitere 30 Minuten behandelt. Dann wurde der Apparat in rund 30 Minuten auf ca. 30 °C abgekühlt, die Gewebeabschnitte entnommen und intensiv bei etwa 60 °C, anschliessend kalt gespült. Nach dem Schleudern wurde vorsichtig trockengebügelt.

Die Remission der Abschnitte wurde mit dem Remissionsspektralphotometer DMC 25 gemessen und die Weißgrade nach Ganz errechnet.

Beispiel 2

Im Laborfärbeappaat wurden 10 g Polyamid-6-Gewebe gebleicht und gleichzeitig aufgehellt mit den in Tabelle 3 aufgeführten Verbindungen. Die erhaltenen Weißgrade sind in Tabelle 3 den Ergebnissen aus Beispiel 1 zugeordnet. Es zeigte sich, daß die erfindungsgemäßen Substanzen chloritbeständig sind. Durchweg werden die Weißgrade aus Beispiel 1 übertroffen.

Flottenzusammensetzung:

Flotte 1:20
0.25 % Optischer Aufheller
1,25 g/l Natriumchlorit (100 %ig)
1,2 g/l Natriumnitrat
1,2 g/l Bleichhilfsmittel
2,0 g/l Kaliumhydrogentartrat
0,5 g/l Nonylphenoloxethylat

Die Behandlung der Gewebeproben, insbesondere die Temperaturführung, erfolgte auf die gleiche Weise wie in Beispiel 1 angegeben. Die aus den Remissionswerten abgeleiten Weißgrade sind in Tabelle 3 angegeben.

Tabelle 3

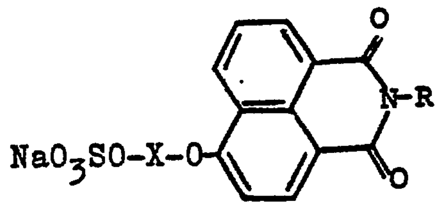

| Nr. | R | X | Weißgrad (WG) ohne Chlorit WG | Nuance | mit Chlorit WG | Nuance |
|---|---|---|---|---|---|---|
| (31) | $-CH_3$ | $-CH_2CH_2-$ | 207 | 0,7 G | 224 | -0,1 B |
| (32) | $-(CH_2)_3CH_3$ | $-CH_2CH_2-$ | 212 | 0,4 B | 227 | -0,1 B |
| (34) | $-CH_2-C_6H_5$ | $-CH_2CH_2-$ | 201 | 1,1 G | 221 | 0,7 G |
| (36) | $-CH_3$ | $-(CH_2)_3-$ | 218 | 0,9 G | 229 | 0,4 G |
| (37) | $-CH_3$ | $-(CH_2)_4-$ | 217 | 1,0 G | 234 | 0,6 G |
| (38) | $-CH_3$ | $-CH_2C(CH_3)_2CH_2-$ | 213 | 1,0 G | 226 | 0,1 B |

## Patentansprüche

1. Verbindungen der allgemeinen Formel (1),

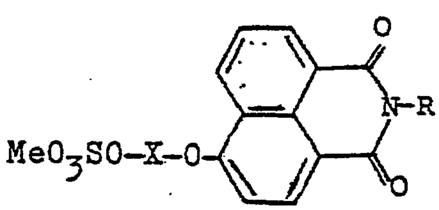

(1)

worin R Wasserstoff, $C_1$–$C_6$-Alkyl, $C_1$–$C_6$-Alkoxy-$C_1$–$C_6$-alkyl, Cyclohexyl, Methylcyclohexyl, Benzyl, Phenylethyl, Phenyl, $C_1$–$C_6$-Alkylphenyl, Xylyl, X eine $C_2$–$C_7$-Alkylengruppe oder ein Brückenglied der Formel $-X^1-(OX^2)_n-$, worin $X^1$ und $X^2$ $C_2H_4$ oder $C_3H_7$ und n 1 oder 2 bedeuten und Me ein Proton, ein Alkalimetallkation oder ein $NH_2R^1R^2$Kation, wobei $R^1$ und $R^2$ für Wasserstoff, $C_1$–$C_6$-Alkyl oder Hydroxy-$C_1$–$C_6$-Alkyl stehen, bedeuten.

2. Verbindungen der Formel 1 nach Anspruch 1, worin R Wasserstoff, $C_1$–$C_4$-Alkyl oder Benzyl, X $C_2$–$C_5$-Alkylen und Me ein Proton, ein Alkalimetallkation oder ein $-NH_2R^1R^2$ Kation, wobei $R^1$ und $R^2$ für Wasserstoff $C_1$–$C_2$-Alkyl oder $C_1$–$C_2$-Hydroxyalkyl stehen, bedeuten.

3. Verfahren zur Herstellung der Verbindung der Formel 1 nach Anspruch 1, dadurch gekennzeichnet, daß man eine Hydroxyverbindung der Formel

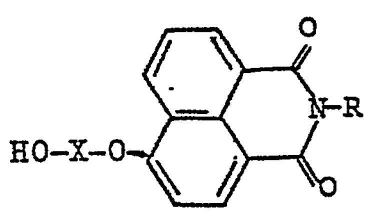

bei 0 bis 30°C mit Schwefelsäure versetzt und 1 bis 4 Stunden bei dieser Temperatur verrührt.

4. Verwendung der Verbindungen nach Anspruch 1 als optische Aufheller.

**Claims**

1. A compound of the formula (1)

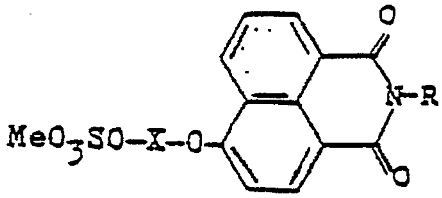

(1)

in which R denotes hydrogen, $C_1$–$C_6$-alkyl, $C_1$–$C_6$-alkoxy-$C_1$–$C_6$-alkyl, cyclohexyl, methylcyclohexyl, benzyl, phenylethyl, phenyl, $C_1$–$C_6$-alkylphenyl or xylyl, X denotes a $C_2$–$C_7$-alkylene group or a bridge member of the formula $-X^1-(OX^2)_n-$ in which $X^1$ and $X^2$ denote $C_2H_4$ or $C_3H_7$ and n denotes 1 or 2 and Me denotes a proton, an alkali metal cation or a cation $NH_2R^1R^2$ in which $R^1$ and $R^2$ represent hydrogen, $C_1$–$C_6$-alkyl or hydroxy-$C_1$–$C_6$-alkyl.

2. A compound of the formula 1 as claimed in claim 1, in which R denotes hydrogen, $C_1$–$C_4$-alkyl or benzyl, X denotes $C_2$–$C_5$-alkylene and Me denotes a proton, an alkali metal cation or a cation $-NH_2R^1R^2$ in which $R^1$ and $R^2$ represent hydrogen, $C_1$–$C_2$-alkyl or $C_1$–$C_2$-hydroxyalkyl.

3. A process for the preparation of a compound of the formula 1 as claimed in claim 1, which comprises adding sulfuric acid to a hydroxyl compound of the formula

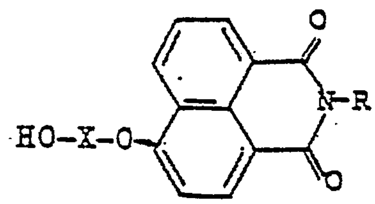

at from 0 to 30°C and stirring the mixture for 1 to 4 hours at this temperature.

4. The use of a compound as claimed in claim 1 as an optical brightener.

**Revendications**

1. Composés répondant à la formule 1:

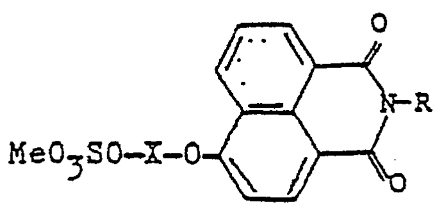

(1)

dans laquelle R représente l'hydrogène, un alkyle en $C_1$–$C_6$, un $C_1$–$C_6$-alcoxy-$C_1$–$C_6$-alkyle, un cyclohexyle, un méthylcyclohexyle, un benzyle, un phényléthyle, un phényle, un $C_1$–$C_6$-alkyl-phényle ou un xylyle, X représente un radical alkylène en $C_2$–$C_7$ ou un radical pont de formule $-X^1-(OX^2)_n-$ dans lequel $X^1$ et $X^2$ représentent chacun $C_2H_4$ ou $C_3H_7$ et n est égal à 1 ou à 2, et Me représente un proton, un cation de métal alcalin ou un cation $-NH_2R^1R^2$ dans lequel $R^1$ et $R^2$ représentent chacun l'hydrogène, un alkyle en $C_1$-$C_6$ ou un hydroxyalkyle en $C_1$–$C_6$.

2. Composés de formule 1 selon la revendication 1 dans lesquels R représente l'hydrogène, un alkyle en $C_1$–$C_4$ ou un benzyle, X représente un alkylène en $C_2$–$C_5$ et Me représente un proton, un cation de métal alcalin ou un cation $-NH_2R^1R^2$ dans lequel $R^1$ et $R^2$ représentent chacun l'hydrogène, un alkyle en $C_1$ ou $C_2$ ou un hydroxyalkyle en $C_1$ ou $C_2$.

3. Procédé pour préparer un composé de formule 1 selon la revendication 1, procédé caractérisé en ce qu'on mélange un composé hydroxylique de formule:

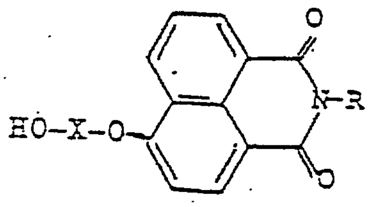

avec de l'acide sulfurique, à une température de 0 à 30°C, et on agite à cette température pendant 1 à 4 heures.

4. Application des composés selon la revendication 1 comme azureurs optiques.